# EUROPEAN PATENT APPLICATION

(11) **EP 4 431 104 A1**
(43) Date of publication of application: **18.09.2024**
(21) Application number: 22892718.2
(22) Date of filing: 07.11.2022
(51) Int. Cl.: A61K 35/28, A61P 19/02, A61P 19/08, A61P 29/00, C12N 5/0775

(54) **TREATMENT FOR KNEE OSTEOARTHRITIS USING ADIPOSE TISSUE-DERIVED MESENCHYMAL STEM CELL LINE**

(30) Priority: 11.11.2021 JP 2021184409
(71) Applicant: Keio University, Tokyo, 108-8345 (JP)
(72) Inventor: FUKUDA, Yoshitsugu, Tokyo 160-8582 (JP); NIKI, Yasuo, Tokyo 160-8582 (JP); NAKAMURA, Masaya, Tokyo 160-8582 (JP); MATSUBARA, Yumiko, Tokyo 160-8582 (JP); URUGA, Yukako, Tokyo 160-8582 (JP)
(74) Representative: Hamer, Christopher K.
(86) International application number: PCT/JP2022/041299
(87) International publication number: WO 2023/085219

(57) **Abstract**

An object of the present invention is to provide, for example, a therapeutic agent using a specific mesenchymal stem cell line derived from an adipose tissue (ASCL), the therapeutic agent (i) stably having an excellent therapeutic effect on one or more diseases selected from knee osteoarthritis, traumatic cartilage defect, and osteochondritis dissecans, (ii) loading less physical burden on a patient, (iii) requiring no restriction on patient's hospital visit management, and (iv) having no restriction on the number of treatments. A feature of the present invention is to use a mesenchymal stem cell line derived from an adipose tissue, the mesenchymal stem cell line being produced by a production method comprising: (A) inducing differentiation of one or more cells selected from a stromal vascular fraction of a vertebrate animal adipose tissue comprising a mesenchymal stem cell, an adipose progenitor cell, and a stromal cell into a mature adipocyte; and (B) inducing dedifferentiation of the mature adipocyte obtained in step (A) to obtain a mesenchymal cell line derived from the vertebrate animal adipose tissue.

## Description

### Technical Field

The present invention relates to, for example, a therapeutic agent for one or more diseases selected from knee osteoarthritis, traumatic cartilage defect, and osteochondritis dissecans, the therapeutic agent comprising a specific mesenchymal stem cell line derived from an adipose tissue as an active ingredient.

### Background Art

Adipose tissue-derived mesenchymal stem cells which are cultured and proliferated from adipose tissues are known to participate in tissue regeneration by differentiation or to promote tissue regeneration by secretion of a growth factor. A treatment method of administering adipose tissue-derived mesenchymal stem cells (also called adipose tissue-derived stem cells: ADSCs) by injection to a joint of an osteoarthritis patient has been clinically introduced as regenerative medicine around the world and is also performed in private practice in Japan. For example, patent document 1 states that adipose tissue-derived mesenchymal stem cells can be used in treatment of knee osteoarthritis.

Meanwhile, the present inventors have previously developed a method for treating a wound by administering a platelet-like cell population produced from adipose tissue-derived mesenchymal stem cells to the wound (patent document 2). The present inventors have also developed a novel method for producing a mesenchymal stem cell line derived from an adipose tissue (adipose-derived mesenchymal stem cell line: ASCL) from vertebrate animal adipose tissue-derived mesenchymal stem cells or the like (i.e., a novel cell line establishing method) (patent document 3). The present inventors are pursuing clinical application as to a technique of producing platelet from ASCL.

### Prior Art Documents

### Patent Documents

Patent Document 1: Japanese unexamined Patent Application Publication No. 2017-93431
Patent Document 2: Japanese unexamined Patent Application Publication No. 2019-34917
Patent Document 3: Domestic re-publication No. 2017/094260 of PCT international application

### Summary of the invention

### Object to be Solved by the Invention

In existing treatment by intra-articular injection using adipose tissue-derived mesenchymal stem cells (ADSCs), patients themselves generally serve as donors of the adipose tissue-derived mesenchymal stem cells. This existing treatment method has some problems. The first problem is that a therapeutic effect of ADSCs has a strong tendency to depend on the quality of donor cells, and the resulting therapeutic effect largely varies in degree. The second problem is that collection of an adipose tissue requires physical burden on a patient. The third problem is that since a period for which prepared ADSCs can exert a preferable therapeutic effect is short, there is a restriction on hospital visit management in such a way that a patient needs to visit a hospital when an adipose tissue is collected and for administration within a relatively short time after a lapse of a given period from the collection. The fourth problem is that in collecting an adipose tissue from a patient, this collection is often performed from the abdomen or the like, whereas there is a restriction on the number of collections of an adipose tissue from the same patient.

An object of the present invention is to provide, for example, a therapeutic agent using a specific mesenchymal stem cell line derived from an adipose tissue (ASCL), the therapeutic agent (i) stably having an excellent therapeutic effect on one or more diseases selected from knee osteoarthritis, traumatic cartilage defect, and osteochondritis dissecans, (ii) loading less physical burden on a patient, (iii) requiring no restriction on patient's hospital visit management, and (iv) having no restriction on the number of treatments.

### Means to Solve the Object

The present inventors conducted extensive studies to solve the above problems and found that, for example, in the case of using ASCL, a model rat with osteoarthritis (OA) of the knee has a low IL-6 concentration in serum and in joint fluid, has a low IFP inflammation score and Mankin score, and receives a significantly high pain suppressive effect, as compared with the case of using ADSCs of a conventional method, whereby the present invention was accomplished.

More specifically, the present invention relates to:
[1] a therapeutic agent for one or more diseases selected from knee osteoarthritis, traumatic cartilage defect, and osteochondritis dissecans, the therapeutic agent comprising a mesenchymal stem cell line derived from an adipose tissue as an active ingredient, the mesenchymal stem cell line being produced by a production method comprising the following steps (A) and (B):
   (A) a step of inducing differentiation of one or more cells selected from a stromal vascular fraction of a vertebrate animal adipose tissue comprising a mesenchymal stem cell, an adipose progenitor cell, and a stromal cell into a mature adipocyte; and
   (B) a step of inducing dedifferentiation of the mature adipocyte obtained in step (A) to obtain a mesenchymal cell line derived from the vertebrate animal adipose tissue;
[2] the therapeutic agent according to the above [1], wherein the step of inducing differentiation of one or more cells into a mature adipocyte in step (A) is a step of culturing the one or more cells in basal medium for mesenchymal cell culture comprising one or more adipose cell differentiation inducing substances selected from the group consisting of dexamethasone, isobutylmethylxanthine, insulin, and serum;
[3] the therapeutic agent according to the above [1] or [2], wherein the dedifferentiation induction of the mature adipocyte in step (B) is performing ceiling culture of the mature adipocyte;
[4] the therapeutic agent according to any one of the above [1] to [3], wherein the one or more cells are cells obtained by removing the mature adipocyte from a cell population obtained by treating the vertebrate animal adipose tissue with an enzyme capable of dispersing the vertebrate animal adipose tissue cells;
[5] the therapeutic agent according to the above [4], wherein the enzyme capable of dispersing the vertebrate animal adipose tissue cells is one or more enzymes selected from the group consisting of collagenase, trypsin, caseinase, clostripain, trypsin-EDTA, dispase, thermolysin, pronase, hyaluronidase, pancreatin, elastase, and papain;
[6] the therapeutic agent according to any one of the above [1] to [5], wherein the mesenchymal stem cell line derived from an adipose tissue expresses one or more surface markers selected from the following surface marker group of mesenchymal cells, and does not express one or more surface markers selected from the following surface marker group of blood cells:
   surface marker group of mesenchymal cells: CD13, CD29, CD44, CD71, CD73, CD90, CD105, CD166, HLA-ABC;
   surface marker group of blood cells: CD11b, CD14, CD19, CD34, CD41, CD42b, CD45, CD56, HLA-DR;
[7] the therapeutic agent according to any one of the above [1] to [6], wherein the therapeutic agent is intra-articularly administered to a vertebrate animal in need of treatment of one or more diseases selected from knee osteoarthritis, traumatic cartilage defect, and osteochondritis dissecans; and
[8] the therapeutic agent according to any one of the above [1] to [7], wherein the vertebrate animal is a human.

### Effect of the Invention

The present invention can provide a therapeutic agent using a specific mesenchymal stem cell line derived from an adipose tissue (ASCL), the therapeutic agent (i) stably having an excellent therapeutic effect on one or more diseases selected from knee osteoarthritis, traumatic cartilage defect, and osteochondritis dissecans, (ii) loading less physical burden on a patient, (iii) requiring no restriction on patient's hospital visit management, and (iv) having no restriction on the number of treatments.

### Brief Description of Drawings

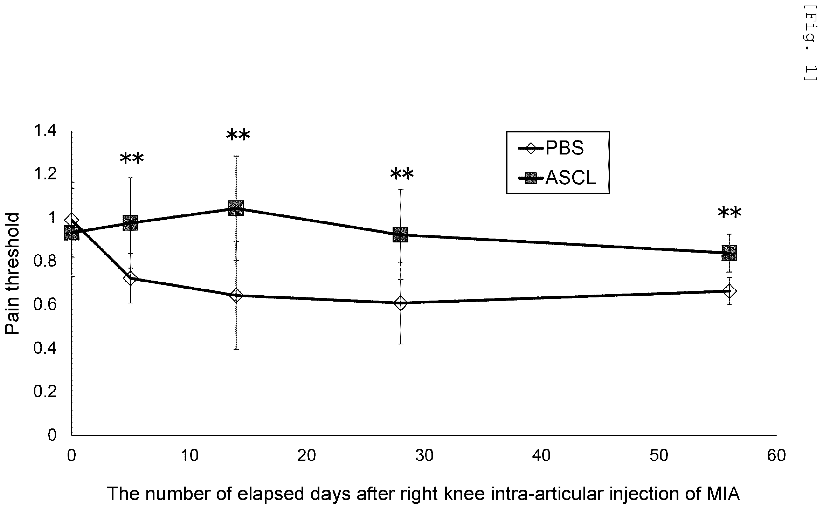

[Figure 1] Figure 1 is a diagram showing results of conducting a mechanical stimulation test using a dynamic plantar aesthesiometer as to MIA-OA model rats of an ASCL treatment group ("ASCL" in the diagram, n = 10) and a control group ("PBS" in the diagram, n = 10) before right knee intra-articular injection of monoiodoacetate (MIA) and on 5, 14, 28, and 56 days after the injection, and measuring pain thresholds. "**" in the diagram represents statistically significant difference from the control group (p < 0.01) (the same holds true for the description below).

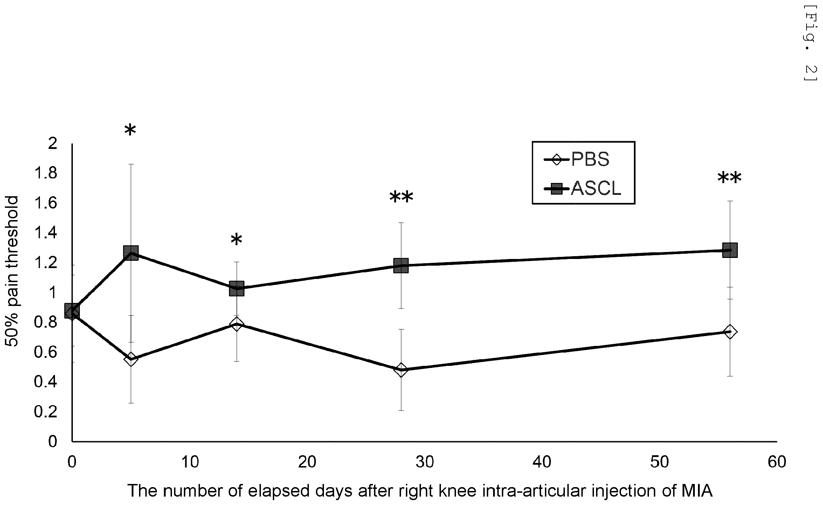

[Figure 2] Figure 2 is a diagram showing results of conducting a mechanical stimulation test by an up-down method using Von Frey monofilaments as to MIA-OA model rats of an ASCL treatment group ("ASCL" in the diagram, n = 10) and a control group ("PBS" in the diagram, n = 10) before right knee intra-articular injection of MIA and on 5, 14, 28, and 56 days after the injection, and measuring 50% pain thresholds. "*" in the diagram represents statistically significant difference from the control group (p < 0.05) (the same holds true for the description below).

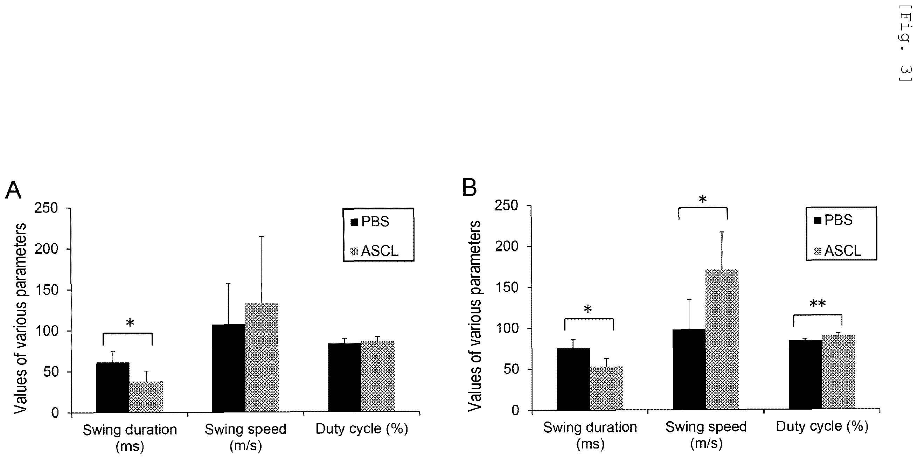

[Figure 3] Figure 3 is a diagram showing results of conducting gait analysis using three types of gait parameters (swing duration (s), swing speed (m/s), and duty cycle (%)) as to MIA-OA model rats of an ASCL treatment group ("ASCL" in the diagram, n = 5) and a control group ("PBS" in the diagram, n = 5) on 14 (Figure 3A) and 56 (Figure 3B) days after right knee intra-articular injection of MIA.

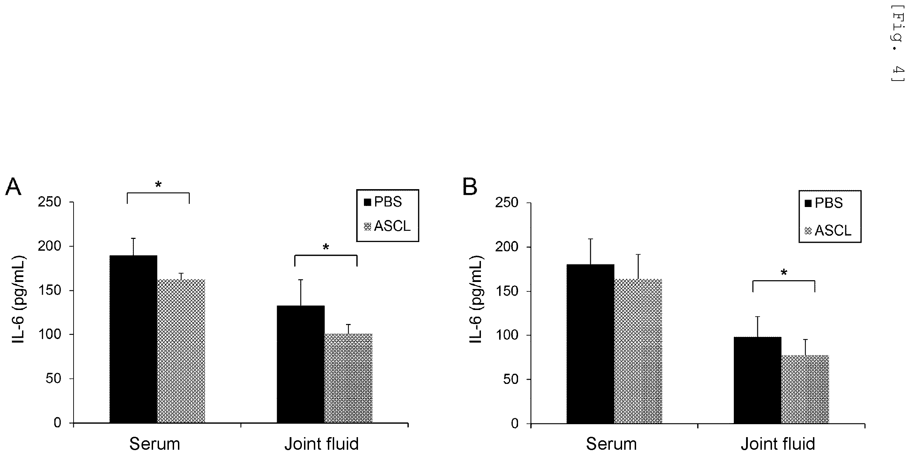

[Figure 4] Figure 4A is a diagram showing results of preparing serum (n = 5 for each group) and joint fluid (n = 5 for each group) from MIA-OA model rats of an ASCL treatment group ("ASCL" in the diagram) and a control group ("PBS" in the diagram) on 5 days after right knee intra-articular injection of MIA, and measuring IL-6 concentrations. Figure 4B is a diagram showing results of preparing serum (n = 10 for each group) and joint fluid (n = 7 for each group) from the MIA-OA model rats of the ASCL treatment group ("ASCL" in the diagram) and the control group ("PBS" in the diagram) on 56 days after right knee intra-articular injection of MIA, and measuring IL-6 concentrations.

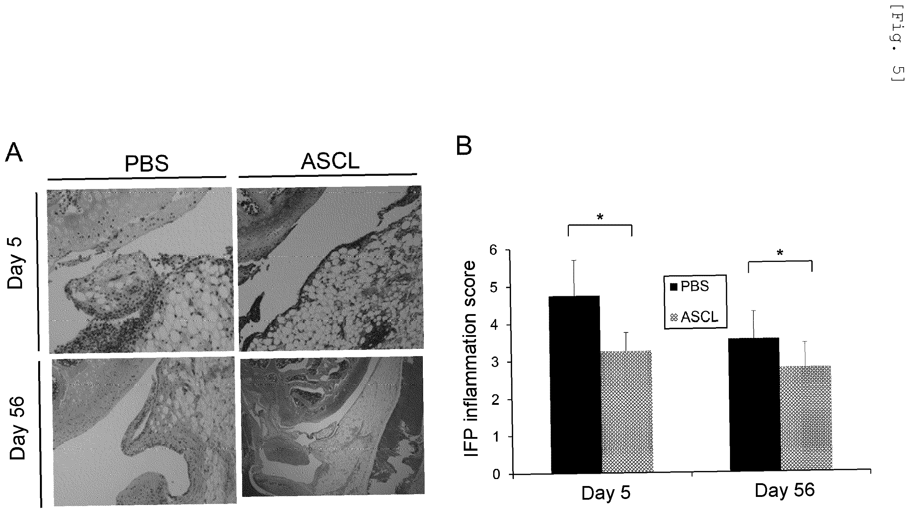

[Figure 5] Figure 5 is a diagram showing results of preparing tissue sections containing synovium and fat pad (Figure 5A) from MIA-OA model rats of an ASCL treatment group ("ASCL" in the diagram) and a control group ("PBS" in the diagram) on 5 (n = 5 for each group) and 56 (n = 10 for each group) days after right knee intra-articular injection of MIA, and evaluating synovitis using IFP (infrapatellar fat pad) inflammation scores (Figure 5B).

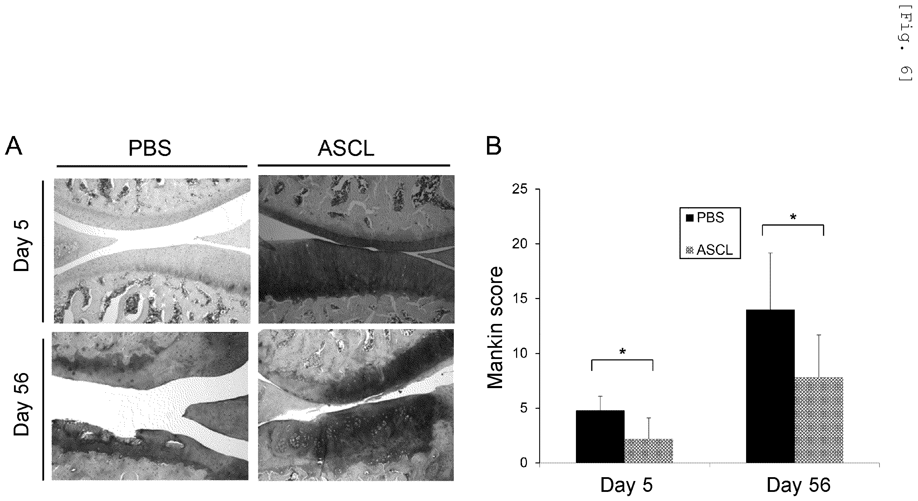

[Figure 6] Figure 6 is a diagram showing results of preparing tissue sections of medial joint space (Figure 6A) from MIA-OA model rats of an ASCL treatment group ("ASCL" in the diagram) and a control group ("PBS" in the diagram) on 5 (n = 5 for each group) and 56 (n = 7 for each group) days after right knee intra-articular injection of MIA, and evaluating cartilage degeneration using Mankin scores (Figure 6B).

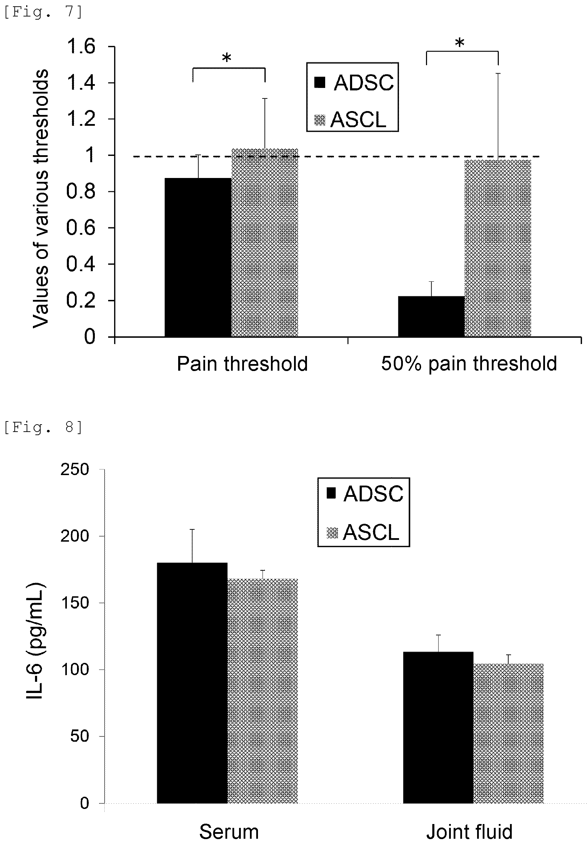

[Figure 7] Figure 7 is a diagram showing results of conducting the same mechanical stimulation test as in Figure 1 and measuring pain thresholds, while conducting the same mechanical stimulation test as in Figure 2 and measuring 50% pain thresholds, as to MIA-OA model rats of an ADSC treatment group ("ADSC" in the diagram, n = 3) and an ASCL treatment group ("ASCL" in the diagram, n = 6) on 5 days after right knee intra-articular injection of MIA. The dotted line in the diagram represents the same state (normal value) as that before administration of MIA.
[Figure 8] Figure 8 is a diagram showing results of preparing serum and joint fluid from MIA-OA model rats of an ADSC treatment group ("ADSC" in the diagram, n = 3) and an ASCL treatment group ("ASCL" in the diagram, n = 6) on 5 days after right knee intra-articular injection of MIA, and measuring IL-6 concentrations.

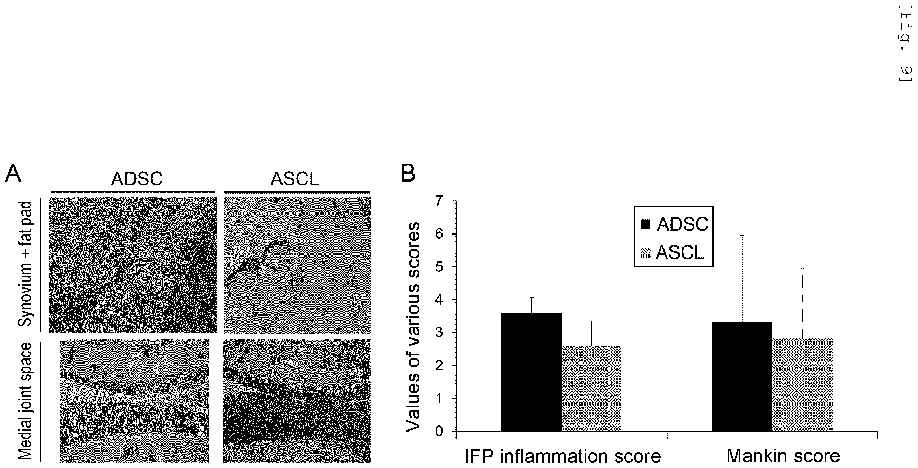

[Figure 9] Figure 9 is a diagram showing results of evaluating synovitis in the same manner as in Figure 5, while evaluating cartilage degeneration in the same manner as in Figure 6, as to MIA-OA model rats of an ADSC treatment group ("ADSC" in the diagram, n = 3) and an ASCL treatment group ("ASCL" in the diagram, n = 6) on 5 days after right knee intra-articular injection of MIA.

### Mode of Carrying Out the Invention

The present invention includes an embodiment of
[1] a therapeutic agent (hereinafter also referred to as "therapeutic agent of the present invention" in the present specification) for one or more diseases selected from knee osteoarthritis, traumatic cartilage defect, and osteochondritis dissecans (hereinafter also simply referred to as "diseases according to the present invention" in the present specification), the therapeutic agent comprising a mesenchymal stem cell line derived from an adipose tissue as an active ingredient, the mesenchymal stem cell line being produced (preferably, having been produced) by a production method (hereinafter referred to as "method for producing a cell line according to the present invention" in the present specification) comprising the following steps (A) and (B) :
   (A) a step of inducing differentiation of one or more cells selected from a stromal vascular fraction of a vertebrate animal adipose tissue comprising a mesenchymal stem cell, an adipose progenitor cell, and a stromal cell into a mature adipocyte; and
   (B) a step of inducing dedifferentiation of the mature adipocyte obtained in step (A) to obtain a mesenchymal cell line derived from the vertebrate animal adipose tissue.

The present invention also includes, as other embodiments, embodiments of:
[2] a method for treating one or more diseases selected from knee osteoarthritis, traumatic cartilage defect, and osteochondritis dissecans, the method comprising the step of administering a mesenchymal stem cell line derived from an adipose tissue to a subject in need of treatment of the diseases, the mesenchymal stem cell line being produced (preferably, having been produced) by a production method comprising the above steps (A) and (B);
[3] a mesenchymal stem cell line derived from an adipose tissue for use in treatment of one or more diseases selected from knee osteoarthritis, traumatic cartilage defect, and osteochondritis dissecans, wherein the mesenchymal stem cell line derived from an adipose tissue is produced (preferably, has been produced) by a production method comprising the above steps (A) and (B);
[4] use of a mesenchymal stem cell line derived from an adipose tissue, in the manufacture of a therapeutic agent for one or more diseases selected from knee osteoarthritis, traumatic cartilage defect, and osteochondritis dissecans, wherein the mesenchymal stem cell line derived from an adipose tissue is produced (preferably, has been produced) by a production method comprising the above steps (A) and (B) ;
[5] a mesenchymal stem cell line derived from an adipose tissue for use as a therapeutic agent for one or more diseases selected from knee osteoarthritis, traumatic cartilage defect, and osteochondritis dissecans, wherein the mesenchymal stem cell line derived from an adipose tissue is produced (preferably, has been produced) by a production method comprising the above steps (A) and (B); etc.

The therapeutic agent of the present invention is not particularly limited as long as the therapeutic agent is a therapeutic agent for one or more diseases selected from knee osteoarthritis, traumatic cartilage defect, and osteochondritis dissecans, the therapeutic agent comprising a mesenchymal stem cell line derived from an adipose tissue as an active ingredient, the mesenchymal stem cell line being produced by a production method comprising the following steps (A) and (B):
(A) a step of inducing differentiation of one or more cells selected from a stromal vascular fraction of a vertebrate animal adipose tissue comprising a mesenchymal stem cell, an adipose progenitor cell, and a stromal cell into a mature adipocyte; and
(B) a step of inducing dedifferentiation of the mature adipocyte obtained in step (A) to obtain a mesenchymal cell line derived from the vertebrate animal adipose tissue.

The mature adipocyte obtained by the differentiation induction in step (A) is a mature adipocyte more easily dedifferentiated (hereinafter also referred to as "easy-to-dedifferentiate mature adipocyte" in the present Description) than a mature adipocyte which have been present in a vertebrate animal adipose tissue, and thus the dedifferentiation induction in step (B) presumably enables the produce (establishment) of a mesenchymal cell line derived from a vertebrate animal adipose tissue (ASCL) more simply, in a shorter period of time, and more efficiently. The present inventors have already confirmed that ASCL produced from the easy-to-dedifferentiate mature adipocyte (i.e., ASCL produced by the production method according to the present invention) differs as a cell from a mesenchymal stem cell line derived from an adipose tissue produced by direct dedifferentiation induction of a mature adipocyte obtained from a vertebrate animal adipose tissue. The "ASCL" as used herein refers to a mesenchymal stem cell line derived from an adipose tissue produced by the production method according to the present invention.

The above production method according to the present invention can be an ex vivo or in vitro production method.

### (Method for producing cell line according to present invention)

The mesenchymal stem cell line derived from an adipose tissue according to the present invention is a cell line produced by the method for producing a cell line according to the present invention. The method for producing a cell line according to the present invention is not particularly limited as long as it is a production method comprising the above steps (A) and (B).

### (Step A)

The above step (A) is not particularly limited as long as it is a step of inducing differentiation of one or more cells selected from a stromal vascular fraction of a vertebrate animal adipose tissue comprising a mesenchymal stem cell, an adipose progenitor cell, and a stromal cell (also referred to as "mesenchymal stem cells" in the present Description) into a mature adipocyte. The step of differentiation induction is an ex vivo or in vitro step of differentiation induction.

The species from which the adipose tissue is derived is not particularly limited as long as a species is a vertebrate animal, and examples include a mammal, a bird, a reptile, an amphibian, and a fish, of which a mammal such as a human, a mouse, a rat, a guinea pig, a rabbit, a cat, a dog, a horse, a cow, a monkey, a sheep, a goat, and a pig being preferable, of which a human being particularly preferable. For a mesenchymal stem cell line derived from a vertebrate animal adipose tissue produced by the method for producing a cell line of the present invention, it is preferable to use the adipose tissue of the target vertebrate animal in the method for producing a cell line of the present invention in light of minimizing a rejection reaction or the like.

The "adipose tissue" as used herein is not particularly limited as long as a tissue comprises fats and examples include a subcutaneous adipose tissue, an adipose tissue in the bone marrow, and a visceral adipose tissue, with a subcutaneous adipose tissue being preferable in light of comparatively low invasiveness to a vertebrate animal supplying the adipose tissue and being comparatively easily collectable.

The "stromal vascular fraction" as used herein means the cells other than mature adipocytes among the cells of a vertebrate animal adipose tissue. A stromal vascular fraction typically comprises cells such as a mesenchymal stem cell, an adipose progenitor cell, a stromal cell, a vascular endothelial cell, a cell related to blood, a smooth muscle cell, and a fibroblast. The "stromal vascular fraction" can be obtained by removing mature adipocytes from a cell population obtained by treating a vertebrate animal adipose tissue with an enzyme capable of dispersing the vertebrate animal adipose tissue cells.

The above "one or more cells selected from a stromal vascular fraction of a vertebrate animal adipose tissue comprising a mesenchymal stem cell, an adipose progenitor cell, and a stromal cell" are not limited as long as one or more cells are selected from a stromal vascular fraction of a vertebrate animal adipose tissue comprising a mesenchymal stem cell, a preadipocyte or an adipose progenitor cell, and a stromal cell, but in light of more efficiently producing a mesenchymal cell line derived from a vertebrate animal adipose tissue, the cell population, rather than comprising only an adipose progenitor cell, preferably comprises at least an adipose progenitor cell and a mesenchymal stem cell and/or a stromal cell, with the cell population comprising at least an adipose progenitor cell, a mesenchymal stem cell, and a stromal cell being more preferable, with the cell population of a stromal vascular fraction being further preferable in light of easy preparation.

Further, examples of the preferable embodiment of the above "one or more cells selected from a stromal vascular fraction of a vertebrate animal adipose tissue comprising a mesenchymal stem cell, an adipose progenitor cell, and a stromal cell" include one or more cells selected from a stromal vascular fraction comprising a mesenchymal stem cell, an adipose progenitor cell, and a stromal cell obtained by dispersing cells of the vertebrate animal adipose tissue, of which a cell population obtained by removing mature adipocytes from a cell population obtained by treating the vertebrate animal adipose tissue with an enzyme capable of dispersing the vertebrate animal adipose tissue cells (cell population A) being preferable. A cell population obtained by further removing vascular endothelial cells and/or cells related to blood from the cell population A may also be used. The above cell population (cell population A) obtained by removing mature adipocytes from a cell population obtained by treating a vertebrate animal adipose tissue with an enzyme capable of dispersing the vertebrate animal adipose tissue cells is a cell population of stromal vascular fractions, which typically comprises cells such as a mesenchymal stem cell, an adipose progenitor cell, a stromal cell, a vascular endothelial cell, a cell related to blood, a smooth muscle cell and a fibroblast of a vertebrate animal adipose tissue.

Examples of the above "treating a vertebrate animal adipose tissue with an enzyme capable of dispersing vertebrate animal adipose tissue cells" include a method in which a vertebrate animal adipose tissue is immersed in a solution comprising such an enzyme and incubated, for example, for about 30 minutes to 3 hours.

The above "enzyme capable of dispersing vertebrate animal adipose tissue cells" is not particularly limited as long as it can disperse cells of a vertebrate animal adipose tissue when allowed to act on the vertebrate animal adipose tissue, and examples include one or more enzymes selected from the group consisting of collagenase, trypsin, caseinase, clostripain, trypsin-EDTA, dispase, thermolysin, pronase, hyaluronidase, pancreatin, elastase, and papain, of which at least one or more enzymes selected from the group consisting of collagenase, trypsin, caseinase, and clostripain being preferable, and commercial collagenase (type I) and collagenase (type II) being more preferable, with collagenase (type II) being further preferable. Further, the above "enzyme capable of dispersing vertebrate animal adipose tissue cells" preferably comprises at least collagenase.

The above "removing mature adipocytes from a cell population obtained by treating a vertebrate animal adipose tissue with an enzyme capable of dispersing the vertebrate animal adipose tissue cells" is not particularly limited as long as a method can remove mature adipocytes from such a cell population, but examples preferably include a method of recovering a cell population (cell pellet) which is precipitated by centrifugation of a suspension comprising the above cell population. Mature adipocytes comprise a large amount of fats, thus have a light specific gravity and float in the upper part of supernatant when centrifuged, hence the recovery of a cell pellet precipitated by the centrifugation enables the removal of mature adipocytes. Further, the method for removing vascular endothelial cells, smooth muscle cells, and fibroblasts from the cell population obtained by treating a vertebrate animal adipose tissue with an enzyme capable of dispersing vertebrate animal adipose tissue cells is not particularly limited as long as a method can remove these cells from such a cell population and examples include a method for removing vascular endothelial cells from the cell population when CD31 known as a surface marker of the vascular endothelial cell selects negative cells (or CD31 removes positive cells), and examples of the method for removing cells related to blood include a method for removing cells related to blood from the cell population by selecting CD45- (a surface marker of hematopoietic cells other than red blood cell and platelet) negative and Ter119- (a surface marker of red blood cell and progenitor cell thereof) negative cells (or CD45-positive and Ter119-positive cells are removed). Additionally, when 7-amino-actinomycin D (7-AAD), which is not a surface marker, being negative is used as an indicator, it is preferable because dead cells comprised in a vertebrate animal adipose tissue can be excluded. 7-AAD intercalates with a DNA chain of a dead cell and produces red fluorescence at a 488-nm excitation light.

The above precipitated cell pellet (cell population A) is cells of a stromal vascular fraction, which typically comprises a mesenchymal stem cell, an adipose progenitor cell, a stromal cell (a stroma cell), a vascular endothelial cell, a smooth muscle cell, and a fibroblast, however, those of which differentiable into a mature adipocyte is a mesenchymal stem cell, an adipose progenitor cell, and a stromal cell. For this reason, a further step may or may not be included for removing any one or more, or all kinds, of the cells other than these 3 kinds from the above precipitated cell pellet before the differentiation induction into a mature adipocyte is carried out, but it is preferable not to include such a step in light of convenience of operation. A vascular endothelial cell, a smooth muscle cell, and a fibroblast do not differentiate into a mature adipocyte even when induced with mesenchymal stem cells to differentiate into a mature adipocyte or do not interfere in the differentiation of mesenchymal stem cells into mature adipocytes.

In the above step (A), examples of the method for inducing differentiation of one or more cells selected from a stromal vascular fraction of a vertebrate animal adipose tissue comprising a mesenchymal stem cell, an adipose progenitor cell, and a stromal cell into a mature adipocyte preferably include a method for culturing the one or more cells selected from a stromal vascular fraction of a vertebrate animal adipose tissue comprising a mesenchymal stem cell, an adipose progenitor cell, and a stromal cell in basal medium for mesenchymal cell culture comprising adipose cell differentiation inducing substances. The method for culturing mesenchymal stem cells in basal medium for mesenchymal cell culture comprising adipose cell differentiation inducing substances is not particularly limited as long as a method can induce differentiation of mesenchymal cells into mature adipocytes by such a culture, and the same method as the typical method for inducing differentiation of an adipose progenitor cell into a mature adipocyte, that is, a method for culturing a starting cell in basal medium for mesenchymal cell culture comprising adipose cell differentiation inducing substances can be used.

In the above step (A), examples of the conditions for culturing mesenchymal stem cells in basal medium for mesenchymal cell culture comprising adipose cell differentiation inducing substances include a method of adhesion culture in a culture vessel coated with the extracellular matrix, and examples of the culture temperature include typically a range from 12 to 45°C, preferably a range from 15 to 37°C, and examples of the culture period include, in light of the balance between producing a mesenchymal cell line derived from a vertebrate animal adipose tissue more efficiently and producing in a shorter period of time, a range from 5 to 16 days, preferably a range from 7 to 14 days, more preferably a range from 8 to 12 days, further preferably a range from 9 to 11, more preferably for 10 days. In the culture, mesenchymal stem cells may or may not be subcultured. Further, examples of the above extracellular matrix include at least one or more components selected from collagen, fibronectin, proteoglycan, and laminin, and BD Matrigel (registered trademark) (manufactured by BD Biosciences) comprising these components can also be used.

The above adipose cell differentiation inducing substances are not particularly limited as long as a substance has an action to differentiate a cell inducible to differentiate into a mature adipocyte or has an assisting action on the action, and examples include one or more substances selected from the group consisting of dexamethasone, isobutylmethylxanthine, insulin, and serum, of which, in light of obtaining a good differentiation inducing efficiency into a mature adipocyte, "combination of serum and dexamethasone", "combinations of adipose cell differentiation inducing substances comprising at least serum and dexamethasone", "combination of serum and isobutylmethylxanthine", and "combinations of adipose cell differentiation inducing substances comprising at least serum and isobutylmethylxanthine" being preferable, of which "combination of serum, dexamethasone, and insulin", "combinations of adipose cell differentiation inducing substances comprising at least serum, dexamethasone, and insulin", "combination of serum, isobutylmethylxanthine, and insulin", "combinations of adipose cell differentiation inducing substances comprising at least serum, isobutylmethylxanthine and insulin", "combination of serum, dexamethasone, and isobutylmethylxanthine", and "combinations of adipose cell differentiation inducing substances comprising at least serum, dexamethasone, and isobutylmethylxanthine" being more preferable, of which "combination of serum, dexamethasone, isobutylmethylxanthine, and insulin", "combinations of adipose cell differentiation inducing substances comprising at least serum, dexamethasone, isobutylmethylxanthine, and insulin" being further preferable. The adipose cell differentiation inducing substances and the basal medium for mesenchymal cell culture comprising such a substance may be commercial products, or a medium prepared by adding an adipose cell differentiation inducing substance to the basal medium for mesenchymal cell culture may be used as such a medium. Examples of the commercial medium comprising an adipose cell differentiation inducing substance preferably include Adipocyte Differentiation Medium (manufactured by Cell Applications, Inc.). Examples of the substance having an assisting action on the action to differentiate into a mature adipocyte other than the above listed adipose cell differentiation inducing substances include Rosiglitazone, Pioglitazone, and Indomethacin.

The concentration of the above adipose cell differentiation inducing substances in the medium is not particularly limited as long as a concentration can induce mesenchymal stem cells into mature adipocytes but examples include, in terms of dexamethasone concentration, typically a range from 0.1 to 10 µM, preferably a range from 0.5 to 2.5 µM, in terms of isobutylmethylxanthine concentration, a range from 10 to 1000 µM, preferably a range from 250 to 750 µM, in terms of insulin concentration, a range from 0.1 to 10 µM, preferably a range from 0.5 to 2.5 µM, and in terms of serum concentration, a range from 1 to 20 wt%, preferably a range from 5 to 15 wt%, more preferably 7 to 13 wt%.

The "basal medium for mesenchymal cell culture" in the present Description is not particularly limited as long as medium can culture at least 1 kind of the mesenchymal cells therein and proliferate the mesenchymal cell, but in light of easy preparation and preventing lot-to-lot variation, a chemically synthesized medium is preferable, and the medium preferably comprises one or more saccharide(s), one or more inorganic salt(s), one or more amino acid(s), and one or more vitamin(s), and one or more other components as needed.

Examples of the above saccharide specifically include a monosaccharide such as glucose, mannose, fructose and galactose, and a disaccharide such as sucrose, maltose, and lactose, of which glucose being particularly preferable, and one or more of these saccharides can be added in combination.

Examples of the above inorganic salt specifically include one or more inorganic salt(s) such as calcium chloride, calcium nitrate, a copper sulfate pentahydrate, an iron(III) nitrate nonahydrate, an iron (II) sulfate heptahydrate, a magnesium chloride hexahydrate, magnesium sulfate, potassium chloride, sodium chloride, sodium bicarbonate, disodium hydrogen phosphate, a disodium hydrogenphosphate dihydrate, sodium dihydrogen phosphate, a sodium dihydrogen phosphate monohydrate, a sodium dihydrogen phosphate dihydrate, a sodium selenite pentahydrate, and a zinc sulfate heptahydrate.

Examples of the above amino acids specifically include one or more amino acid(s) selected from alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, and valine, and preferably include an L-form amino acid and a derivative thereof and a by-product such as a salt thereof and a hydrate thereof. Examples of the above arginine include an arginine by-product such as an L-arginine hydrochloride and an L-arginine monohydrochloride, examples of the above aspartic acid include an aspartic acid by-product such as an L-sodium aspartate monohydrate, an L-aspartic acid monohydrate, potassium L-aspartate, and magnesium L-aspartate, examples of the above cysteine include a cysteine by-product such as L-cysteine dihydrochloride and an L-cysteine hydrochloride monohydrate, and a lysine by-product such as L-lysine hydrochloride, examples of the above glutamic acid include a glutamine by-product such as monosodium L-glutamate, examples of the above asparagine include an asparagine by-product such as an L-asparagine monohydrate, examples of the above tyrosine include a tyrosine by-product such as an L-tyrosine disodium dihydrate, examples of the above histidine include a histidine by-product such as histidine hydrochloride and a histidine hydrochloride monohydrate, and examples of the above lysine include a lysine by-product such as L-lysine hydrochloride.

Examples of the above vitamins specifically include one or more vitamin(s) selected from biotin, choline, folic acid, inositol, niacin, pantothenic acid, pyridoxine, riboflavin, thiamine, vitamin B12, paraaminobenzoic acid (PABA), and ascorbic acid, and a derivative each thereof and a by-product thereof such as a salt thereof and a hydrate thereof. Examples of the above choline include a choline by-product such as choline chloride, examples of the niacin include a niacin by-product such as nicotinic acid, nicotinamide, and nicotinic alcohol, examples of the pantothenic acid include a pantothenic acid by-product such as calcium pantothenate, sodium pantothenate, and panthenol, examples of the pyridoxine include a pyridoxine by-product such as pyridoxine hydrochloride, pyridoxal hydrochloride, pyridoxal phosphate, and pyridoxamine, examples of the thiamine include a thiamine by-product such as thiamine hydrochloride, thiamine nitrate, bisthiamine nitrate, a thiamine dicetyl sulfate ester salt, fursultiamine hydrochloride, octothiamine, and benfotiamine, examples of the ascorbic acid include an ascorbic acid by-product such as ascorbic acid 2-phosphate, ascorbic acid magnesium phosphate, sodium ascorbate sulfate, aminopropyl ascorbyl phosphate, and sodium ascorbate phosphate.

Examples of the above other components include a buffer such as HEPES, an antibiotic such as penicillin and streptomycin, pyruvic acid and a derivative thereof and a by-product thereof such as a salt thereof and a hydrate thereof, and phenol red, examples of the above antibiotics include penicillin G sodium and streptomycin sulfate, or preferably a penicillin-streptomycin solution, and examples of the pyruvic acid by-product preferably include sodium pyruvate.

Specific examples of the above basal medium for mesenchymal cell culture include a known commercial chemically synthesized medium such as Dulbecco's modified Eagle's medium (DMEM), Iscove's Modified Dulbecco's Medium (IMDM), RPMI 1640 medium, minimum essential medium (MEM), basal medium of Eagle (BME), and F12 medium, a medium of 2 or more of these medium mixed in a suitable ratio such as DMEM/F12 (medium of DMEM and F12 medium mixed in 1:1), with medium to which one or more substances selected from the group consisting of antibiotics such as penicillin and streptomycin; and additional amino acids (preferably non-essential amino acids); are added being preferable, and medium wherein an antibiotic (preferably penicillin G sodium, streptomycin sulfate or a penicillin-streptomycin solution) is further added to DMEM, IMDM or RPMI 1640 medium being particularly more preferable, of which medium wherein an antibiotic (preferably penicillin G sodium, streptomycin sulfate, or a penicillin-streptomycin solution) is further added to DMEM being particularly preferable.

Examples of the particularly preferable basal medium for mesenchymal cell culture in the present invention include medium wherein 100 U/mL (final concentration) of a penicillin-streptomycin solution is added to DMEM having the composition to be described later (hereinafter referred to as "particularly preferable basal medium in the present invention"), and medium comprising each component in a concentration of the proportion ranging independently from 70% to 130% to the concentration of each component in the particularly preferable basal medium in the present invention.

### (Composition of DMEM)

200 mg/L of anhydrous calcium chloride, 0.1 mg/L of Fe(NO₃)₃• 9H₂O, 200 mg/L of potassium chloride, 97.67 mg/L of anhydrous magnesium sulfate, 6400 mg/L of sodium chloride, 3700 mg/L of sodium bicarbonate, 125 mg/L of sodium dihydrogen phosphate monohydrate, 4500 mg/L of D-glucose, 15 mg/L of phenol red, 110 mg/L of sodium pyruvate, 84 mg/L of L-arginine hydrochloride, 63 mg/L of L-cysteine dihydrochloride, 584 mg/L of L-glutamine, 30 mg/L of glycine, 42 mg/L of L-histidine hydrochloride monohydrate, 105 mg/L of L-isoleucine, 105 mg/L of L-leucine, 146 mg/L of L-lysine hydrochloride, 30 mg/L of L-methionine, 66 mg/L of L-phenylalanine, 42 mg/L of L-serine, 95 mg/L of L-threonine, 16 mg/L of L-tryptophan, 104 mg/L of L-tyrosine disodium dihydrate, 94 mg/L of L-valine, 4 mg/L of D-calcium pantothenate, 4 mg/L of choline chloride, 4 mg/L of folic acid, 7.2 mg/L of i-inositol, 4 mg/L of nicotinamide, 4 mg/L of pyridoxine hydrochloride, 0.4 mg/L of rivoflavin, 4 mg/L of thiamine hydrochloride.

Mature adipocytes obtained in the above step (A) (that is, the mature adipocyte population comprising mature adipocytes) are easy-to-dedifferentiate mature adipocytes, which are comparatively easily dedifferentiated when induced to dedifferentiate (that is, easy-to-dedifferentiate mature adipocyte population comprising easy-to-dedifferentiate mature adipocytes). The "easy-to-dedifferentiate mature adipocyte population" in the present Description means a mature adipocyte population having a proportion of the cell line obtained which is 1.5 or more times more than that of the mature adipocyte population collected from a vertebrate animal adipose tissue as described in the conventional method (Japanese Patent No. 5055611), and includes mature adipocyte populations having preferably 2 or more times, more preferably 4 or more times, further preferably 6 or more times, more preferably 10 or more times, and further preferably 15 or more times proportions. The above "proportion of the cell line obtained" indicates the proportion of cell line obtained from a specific amount of a mature adipocyte population, and the proportion preferably includes, for example, a proportion (rate) of "a weight of a cell line to be obtained" to "a weight of mature adipocytes to be used for dedifferentiation induction."

### (Step B)

The above step (B) is not particularly limited as long as a step can induce dedifferentiation of the mature adipocytes (easy-to-dedifferentiate mature adipocytes) obtained in step (A) to obtain a mesenchymal cell line derived from a vertebrate animal adipose tissue. The step is an ex vivo or in vitro step.

Mature adipocytes used in step (B) is the mature adipocytes obtained by the differentiation induction in step (A). Such mature adipocytes can be obtained by, for example, centrifuging the culture suspension of step (A) and collecting the cells which float in the upper part of the supernatant. This is because mature adipocytes comprise a large amount of fats, thus have a light specific gravity and float in the upper part of supernatant when centrifuged.

In the above step (B), the method of inducing differentiation of the mature adipocytes (easy-to-dedifferentiate mature adipocytes) obtained in step (A) to obtain a mesenchymal cell line derived from a vertebrate animal adipose tissue is not particularly limited as long as a method can induce differentiation of the mature adipocytes to obtain a mesenchymal cell line derived from a vertebrate animal adipose tissue, but examples preferably include a method of so-called ceiling culture of the mature adipocytes. The ceiling culture is a method for culturing cells by allowing the cells to adhere or float (preferably allowed to adhere) to the inner upper surface (ceiling surface) of a culture vessel (preferably a culture flask) filled up with medium, and this method for culturing cells utilizes the property of mature adipocytes which comprise a large amount of fats, thus have a light specific gravity and float in the medium.

Examples of the medium when carrying out the dedifferentiation inducing culture of a mature adipocyte include basal medium for mesenchymal cell culture comprising the extracellular matrix, and examples of the extracellular matrix include one or more components selected from collagen, fibronectin, proteoglycan, laminin, and serum (FBS), and BD Matrigel (registered trademark) (manufactured by BD Biosciences) comprising such a component can also be used. Serum such as FBS in medium when carrying out the dedifferentiation inducing culture of a mature adipocyte may be used only as an adhesion factor for allowing a mature adipocyte to adhere to the ceiling surface of a culture vessel or may not be used only as the adhesion factor for that purpose. The medium when carrying out the dedifferentiation inducing culture of a mature adipocyte may not comprise serum such as FBS, but in light of producing a mesenchymal cell line derived from a vertebrate animal adipose tissue more efficiently, it is preferable to comprise serum such as FBS with the extracellular matrix other than serum or without the extracellular matrix other than serum. The serum concentration, in the case where the medium comprises serum such as FBS, is not particularly limited as long as a mesenchymal cell line derived from a vertebrate animal adipose tissue is obtained but examples include a range from 3 to 30 wt%, preferably include a range from 7 to 25 wt%, more preferably include a range from 7 to 13 wt%.

In the above step (B), when the conditions, other than the ceiling culture conditions, for culturing a mature adipocyte in the basal medium for mesenchymal cell culture comprising the extracellular matrix are described, examples of the culture temperature typically include a range from 12 to 45°C, preferably a range from 15 to 37°C, and examples of the culture period include a range from 2 to 28 days, preferably a range from 4 to 21 days, more preferably a range from 5 to 14 days, further preferably a range from 6 to 10 days, more preferably for 7 days, in light of balancing the production of a mesenchymal cell line derived from a vertebrate animal adipose tissue between more efficiently and in a shorter period of time. In the culture, a mature adipocyte may or may not be subcultured.

A mesenchymal cell line derived from a vertebrate animal adipose tissue may or may not be isolated from the medium after the ceiling culture in the above step (B), but it is preferable to isolate. When the ceiling culture is continued, mature adipocytes gradually decrease while the established mesenchymal cell line derived from an adipose tissue actively proliferates and a cell population comprising a large amount of mesenchymal cell lines derived from an adipose tissue can be obtained. For example, when the ceiling culture is continued for about 14 days, a cell population containing an extremely large amount of mesenchymal cell lines derived from an adipose tissue can be obtained.

The ceiling culture in the above step (B) include, for the sake of convenience, the continuation of culture in which after the mature adipocyte (easy-to-dedifferentiate mature adipocyte) obtained in step (A) is adhered to the ceiling surface of a culture vessel, the culture vessel is arranged in such a way that the adhesion surface turns to the bottom side thereof, however, the culture may be continued while the mature adipocyte (easy-to-dedifferentiate mature adipocyte) obtained in step (A) are adhered to the ceiling surface of a culture vessel to obtain a mesenchymal cell derived from an adipose tissue without carrying out the culture in which the culture vessel is arranged in such a way that the adhesion surface turns to the bottom side of the medium.

### (Mesenchymal stem cell line derived from adipose tissue according to present invention)

The mesenchymal stem cell line derived from an adipose tissue (ASCL) according to the present invention is not particularly limited as long as it is a mesenchymal stem cell line derived from a vertebrate animal adipose tissue produced by the method for producing a cell line according to the present invention. ASCL owned by AdipoSeeds K.K. is particularly preferable.

The ASCL according to the present invention does not spontaneously differentiate when cultured in the typical basal medium for mesenchymal cell culture which does not have a differentiation inducing action, is suitable for long-term subculture, and maintains proliferation potency and differentiation potency into mesodermal cells (one or more cells selected from the group consisting of a megakaryocyte/platelet, an osteoblast, a cartilage, and an adipocyte) even after long-term subculture. For example, The ASCL according to the present invention produced from a human subcutaneous adipose tissue has been observed to maintain proliferation potency even in the 20th generation and have a doubling time of 23 hours.

The ASCL according to the present invention preferably expresses one or more (preferably 3 or more, more preferably 5 or more, further preferably 7 or more, more preferably 8 or 9, most preferably 9) surface markers selected from the following surface marker group of mesenchymal cells, and does not express one or more (preferably 3 or more, more preferably 5 or more, further preferably 7 or more, more preferably 8 or 9, most preferably 9) surface markers selected from the following surface marker group of blood cells.
Surface marker group of mesenchymal cells: CD13, CD29, CD44, CD71, CD73, CD90, CD105, CD166, HLA-ABC;
Surface marker group of blood cells: CD11b, CD14, CD19, CD34, CD41, CD42b, CD45, CD56, HLA-DR;

International Society for Cellular Therapy sets conditions to define the mesenchymal stem cell as (A) to be an adherent cell, (B) to be capable of differentiating into bones, cartilages, and fats, and (C) to express surface markers of mesenchymal cells and not express surface markers of blood cells. Of the ASCLs according to the present invention, the cell lines of a preferable embodiment meet the conditions (A), (B), and (C).

The ASCL according to the present invention can be proliferated by culture in a mesenchymal stem cell proliferation medium. A commercially available product can be used as the mesenchymal stem cell proliferation medium.

The ASCL according to the present invention is preferably ASCL stimulated with calcium in light of obtaining a better therapeutic effect on one or more diseases selected from knee osteoarthritis, traumatic cartilage defect, and osteochondritis dissecans. Examples of a method for the calcium stimulation include a method of contacting (preferably, dipping) ASCL with an isotonic solution containing a compound containing calcium (for example, one or more compounds selected from the group consisting of calcium chloride, calcium citrate, calcium carbonate, calcium phosphate, calcium sulfate, calcium lactate, and calcium hydroxide). The contact time is not particularly limited, but is, for example, 5 minutes to 1 hour, preferably 10 to 20 minutes. The calcium concentration in the above isotonic solution is, for example, 1 to 100 mM, preferably 5 to 50 mM, more preferably 5 to 20 mM. The method of a particularly preferable embodiment involves stimulating ASCL by contact (preferably, dipping) with an isotonic solution containing 10 mM CaCl₂ for 15 minutes.

### (Application of ASCL according to present invention)

The mesenchymal stem cell line derived from an adipose tissue (ASCL) according to the present invention can be used in treatment of one or more diseases selected from knee osteoarthritis, traumatic cartilage defect, and osteochondritis dissecans (diseases according to the present invention).

The therapeutic agent of the present invention is not particularly limited as long as it contains the mesenchymal stem cell line derived from an adipose tissue (ASCL) according to the present invention as an active ingredient. The therapeutic agent of the present invention is capable of being prepared, stored, and administered with reference to findings about conventionally known cell preparations and in the same manner as therein. The therapeutic agent of the present invention usually has an injectable form. The therapeutic agent of the present invention can be administered into a diseased joint (for example, knee joint) in the target vertebrate animal (preferably, human patient) .

The therapeutic agent of the present invention can be prepared into a preparation in a form suitable for administration to a subject, for example, by mixing the ASCL according to the present invention with a pharmaceutically acceptable carrier. Examples of the carrier include physiological saline, and injectable distilled water rendered isotonic by the addition of glucose or other aids (for example, D-sorbitol, D-mannitol, and sodium chloride). The therapeutic agent of the present invention may be further supplemented with a buffer (for example, a phosphate buffer solution and a sodium acetate buffer solution), a soothing agent (e.g., benzalkonium chloride and procaine hydrochloride), a stabilizer (e.g., human serum albumin and polyethylene glycol), a preservative, an antioxidant, or the like.

The therapeutic agent of the present invention may optionally further comprise, in addition to the ASCL according to the present invention, various additive components generally used and other drugs effective for treatment of the diseases according to the present invention.

The therapeutic target in the treatment of the diseases according to the present invention is not particularly limited as long as it is a vertebrate animal. Examples thereof include a mammal, a bird, a reptile, an amphibian, and a fish, of which a mammal such as a human, a mouse, a rat, a guinea pig, a rabbit, a cat, a dog, a horse, a cow, a monkey, a sheep, a goat, and a pig being preferable, of which a human being particularly preferable.

In the present specification, the treatment of one or more diseases selected from knee osteoarthritis, traumatic cartilage defect, and osteochondritis dissecans (diseases according to the present invention) includes not only improvement in symptoms of the diseases according to the present invention but suppression or delay of worsening of symptoms of the diseases according to the present invention, for example.

The concentration of the ASCL contained in the therapeutic agent of the present invention can be appropriately determined depending on the dose range of the ASCL, the number of medications, etc. Examples thereof include 1 × 10⁵ cells/mL to 5 × 10⁸ cells/mL and 1 × 10⁶ cells/mL to 2 × 10⁸ cells/mL.

The dose range of the ASCL is not particularly limited and may be appropriately set according to the type of the recipient vertebrate animal, the type of a disease, the properties (body weight, age, medical condition, and the presence or absence of use of other drugs, etc.) of the recipient, and the decision of a doctor in charge, etc.

When the therapeutic target in the treatment of the diseases according to the present invention is a human, the number of cells of the ASCL to be administered per dose for one joint is, for example, 1 × 10⁶ or more cells, preferably 5 × 10⁶ or more cells, more preferably 1 × 10⁷ or more cells, further preferably 2 × 10⁷ or more cells, as the lower limit, and is, for example, 2 × 10⁸ or less cells or 1 × 10⁸ or less cells as the upper limit. When the therapeutic target is a non-human vertebrate animal, the dose (the number of cells) of ASCL appropriate for the body weight (kg) of the therapeutic target vertebrate animal can be calculated as a guideline on the basis of a numeric value obtained by converting the number of ASCL listed above to a value per kg body weight with the human body weight defined as 70 kg.

The therapeutic agent of the present invention may be administered once or may be administered twice or more, per joint of the target vertebrate animal. In the case of administering the therapeutic agent of the present invention twice or more, the dosing interval from previous administration can be appropriately determined according to articular symptoms of the target vertebrate animal. Examples thereof include an interval within the range of 3 weeks to 6 weeks and an interval within the range of 6 months to 1 year.

Hereinafter, the present invention will be described in detail with reference to Examples. However, the present invention is not limited by these examples.

### Examples

### 1. Material and method

### 1-1 ASCL

A mesenchymal stem cell line derived from an adipocyte (ASCL; adipose-derived mesenchymal stem cell line) was prepared in accordance with the method described in Examples of International Publication No. WO 2017/094260. Adipose-derived stem cells (ADSCs; adipose tissue-derived stem cells) were obtained from Cell Applications, Inc.

### 1-2 Laboratory animal

A total of 32 T cell function-deficient rats of F344/NJCl-rnu/rnu immunodeficient models (8 weeks old, average body weight: 179.9 ± 0.3 g, male) (hereinafter simply referred to as "rats") were obtained as laboratory animals from CLEA Japan, Inc. The rats were housed at a ratio of two rats per cage and raised by free ingestion of standard diet and tap water in a room having a 12-hour light/dark cycle under a temperature condition of 21 to 22°C.

### 1-3 Intra-articular injection of various stem cells to MIA-OA model rat

Model rats with knee osteoarthritis (OA) induced by monoiodoacetate (MIA) (referred to as "MIA-OA model rats" in the present specification) were prepared by administering 0.2 mg/50 µL of MIA (manufactured by Sigma-Aldrich) to right knee joints of 8-week-old rats by injection in accordance with the method described in the document "Fernihough J, et al., Pain. 2004; 112: 83-93". On the next day, 2.5 × 10⁶ cells/50 µL of ASCL were intra-articularly (diseased side) administered to the right knee for the MIA-OA model rats of an ASCL treatment group; 2.5 × 10⁶ cells/50 µL of ASCL were intra-articularly (diseased side) administered for the MIA-OA model rats of an ADSC treatment group; and the same amount as above (50 pL) of PBS was intra-articularly (diseased side) administered for the MIA-OA model rats of a control group.

### 1-4 Nociception analysis

The presence or absence of nociception was evaluated by two types of mechanical stimulation tests (a mechanical stimulation test using a dynamic plantar aesthesiometer [manufactured by Ugo Basile S.R.L.] and a mechanical stimulation test by the up-down method using Von Frey monofilaments). The mechanical stimulation test using a dynamic plantar aesthesiometer was conducted in accordance with standardized procedures for mechanical stimulation threshold evaluation. Specifically, each MIA-OA model rat was placed in a wire-bottomed transparent plastic case and acclimatized for about 15 minutes. In order to test the center of plantar surface of a hind paw, a steel bar of 0.5 mm in diameter was pressed against the hind paw by an uplifting force of 0 to 50 g at a rate of 2.5 g/sec over 20 seconds using an automatic testing apparatus (dynamic plantar aesthesiometer; manufactured by Ugo Basile S.R.L.). When the MIA-OA model rat withdrew its hind paw, the mechanical stimulation stopped automatically. The paw withdrawal force of the rat was recorded by 0.1 g, and an average value of the force for the right paw (diseased side) with respect to the force for the left hind paw (healthy side) was calculated as a pain threshold (ordinate of Figure 1). The test was continuously conducted three times at intervals of at least 3 to 5 minutes between runs. The mechanical stimulation test by the up-down method using Von Frey monofilaments was conducted in accordance with standardized procedures for mechanical stimulation threshold evaluation. Specifically, each MIA-OA model rat was placed in a wire-bottomed transparent plastic case and acclimatized for about 15 minutes. In order to test the center of plantar surface of a hind paw, filaments (Semmes Weinstein; manufactured by Ugo Basile S.R.L.) having 9 types of stimulus weights (buckling weights) (0.4, 0.6, 1, 2, 4, 6, 8, 10, and 15 g) were used. First, the filament having a buckling weight of 2 g was applied to plantar surface of paws on the same side at a buckling pressure. Pow lifting (escape reaction) for 10 seconds was assessed as a positive response, and the same evaluation was conducted using the filament having a lighter buckling weight. On the other hand, if no positive response was found, this was assessed as a negative response and the same evaluation was conducted using the filament having a heavier buckling weight. A 50% withdrawal (reaction) threshold (g) was determined as a 50% pain threshold (ordinate of Figure 2) by use of the up-down method.

### 1-5 Gait analysis

Gait analysis was conducted using DigiGait, a gait analyzer for a small animal (manufactured by Mouse Specifics, Inc.) in accordance with the method described in the document "Neurosci. Bull. June, 2019, 35 (3): 401-418". The DigiGait is equipped with a transparent treadmill, and each MIA-OA model rat is confined below a polymethyl methacrylate cover and forced to walk/run at a given speed (usually, 5 cm/sec to 20 cm/sec) and gradient (usually, 0%). Each rat was repetitively placed on the treadmill at intervals of at least 5 minutes, and three runs were completed with no break (at least three-step cycle for each run). In this experiment, the speed of the treadmill was set to 10 cm/sec, and the gait analysis employed three types of gait parameters (swing duration (s), swing speed (m/s), and duty cycle (%)) and involved evaluation using Digigait software (see the document "Phytother. Res. 26: 1278-1285 (2012) ") . Here, the "swing duration (s)" means the time during which feet are not in contact with the ground by a complete step cycle; the "swing speed (m/s)" means a value calculated by dividing the "stride length" by the "swing duration"; the "duty cycle (%)" means the ratio between the "stride duration" and the "step cycle duration (s)". The "step cycle duration (s)" means a value obtained by summating the "stride duration" and the "swing duration".

### 1-6 Measurement of inflammatory cytokine

In order to prepare the serum of the MIA-OA model rats, blood collected from the heart was centrifuged and purified in accordance with a standard method. In order to prepare the joint fluid (synovial fluid) of the MIA-OA model rats, the inside of a joint was washed using 100 µL of PBS, followed by recovery. The concentrations of IL-6 (inflammatory cytokine) in the serum and in the joint fluid were measured using an ELISA kit (Quantikine; manufactured by R&D Systems, Inc.).

### 1-7 Evaluation of synovitis and cartilage degeneration

Knee tissues collected from the MIA-OA model rats were sliced at a thickness of 5 pm, defatted with xylene, dehydrated by alcohol gradient, and stained with safranine O. Synovitis was evaluated on the basis of IFP inflammation scores (0 to 6) using tissue sections containing synovium and fat pad in accordance with the method described in the document "M. Udo, et al. Monoiodoacetic acid induces arthritis and synovitis in rats in a dose- and time-dependent manner: proposed model-specific scoring systems Osteoarthritis and Cartilage (2016)". Cartilage degeneration was evaluated on the basis of Mankin scores (0 to 14) using tissue sections of medial joint space in accordance with the method described in the document "Pauli C, et al. Comparison of cartilage histopathology assessment systems on human knee joints at all stages of osteoarthritis development. Osteoarthritis Cartilage (2012) 20: 476-85".

### 2. Results

### 2-1 MIA-OA model rat

First, symptoms of right knee joints (diseased side) of the MIA-OA model rats were confirmed. When 0.2 mg of MIA was administered by injection to the knee joint of each rat, cartilage erosion progressed slowly over 56 days and synovitis was sustained. On 56 days after administration, the cartilage was eroded up to subchondral bone. These results indicate that the MIA-OA model rats reproduced the early and advanced stages of OA.

### 2-2 Nociception analysis

Next, in order to confirm a therapeutic effect of ASCL on OA, nociception analysis was conducted by use of two types of mechanical stimulation tests (a mechanical stimulation test using a dynamic plantar aesthesiometer and a mechanical stimulation test by the up-down method using Von Frey monofilaments). As a result of conducting the mechanical stimulation test using a dynamic plantar aesthesiometer, the pain threshold of the MIA-OA model rats of the control group was constantly lower than that before administration of MIA (see "PBS" in Figure 1), whereas the pain threshold of the MIA-OA model rats of the ASCL treatment group was significantly elevated as compared with the MIA-OA model rats of the control group, and restored to the same level as that before administration of MIA (see "ASCL" in Figure 1). In the case of conducting the mechanical stimulation test by the up-down method using Von Frey monofilaments, the 50% pain threshold was also significantly elevated in the MIA-OA model rats of the ASCL treatment group compared with the MIA-OA model rats of the control group (see Figure 2).

These results indicate that ASCL, when administered to the MIA-OA model rats, can improve pain associated with OA at both the early and advanced stages of OA.

### 2-3 Gait analysis

Next, in order to confirm a therapeutic effect of ASCL on OA, three types of gait parameters (swing duration (s), swing speed (m/s), and duty cycle (%)) were evaluated using DigiGait. As a result, all the values of the gait parameters exhibited an improvement tendency in the MIA-OA model rats of the ASCL treatment group compared with the MIA-OA model rats of the control group (see Figure 3). Particularly, the "swing duration (s)" was significantly shorter in the MIA-OA model rats of the ASCL treatment group than in the MIA-OA model rats of the control group on both 14 (Figure 3A) and 56 (Figure 3B) days after right knee intra-articular injection of MIA. The "swing speed (m/s)" and the "duty cycle (%)" were significantly larger in the MIA-OA model rats of the ASCL treatment group than in the MIA-OA model rats of the control group on 56 days after right knee intra-articular injection of MIA.

These results indicate that ASCL, when administered to the MIA-OA model rats, can improve abnormal gait associated with OA at both the early and advanced stages of OA.

### 2-4 Measurement of inflammatory cytokine

Since OA is an inflammatory disease, relationship between a therapeutic effect of ASCL on OA and an expression level of an inflammatory cytokine was analyzed. As a result, the concentrations of IL6 in serum and in joint fluid were lower in the MIA-OA model rats of the ASCL treatment group than in the MIA-OA model rats of the control group on both 5 and 56 days after right knee intra-articular injection of MIA (see Figure 4). Particularly, the concentrations of IL6 in serum and in joint fluid were significantly lower in the MIA-OA model rats of the ASCL treatment group than in the MIA-OA model rats of the control group on both 5 (Figure 4A) and 56 (Figure 4B) days after right knee intra-articular injection of MIA, and the concentrations of IL6 in serum and in joint fluid were also significantly lower in the MIA-OA model rats of the ASCL treatment group than in the MIA-OA model rats of the control group on 56 days after right knee intra-articular injection of MIA.

These results indicate that ASCL, when administered to the MIA-OA model rats, can suppress increase in inflammatory cytokine level associated with OA at both the early and advanced stages of OA.

### 2-5 Evaluation of synovitis and cartilage degeneration

Next, a therapeutic effect of ASCL on OA was histopathologically analyzed. As a result of evaluating synovitis using an IFP inflammation score, the IFP inflammation score was significantly lower in the MIA-OA model rats of the ASCL treatment group than in the MIA-OA model rats of the control group on both 5 and 56 days after right knee intra-articular injection of MIA (see Figure 5).

These results indicate that ASCL, when administered to the MIA-OA model rats, can suppress synovitis associated with OA at both the early and advanced stages of OA.

As a result of evaluating cartilage degeneration using a Mankin score, the Mankin score was significantly lower in the MIA-OA model rats of the ASCL treatment group than in the MIA-OA model rats of the control group on both 5 and 56 days after right knee intra-articular injection of MIA (see Figure 6).

These results indicate that ASCL, when administered to the MIA-OA model rats, can suppress cartilage degeneration associated with OA at both the early and advanced stages of OA.

### 2-6 Comparison with conventional method

In order to verify whether the therapeutic effect of ASCL on OA was better than that of OA treatment using ADSCs as a conventional method, the MIA-OA model rats of the ASCL treatment group and the ADSC treatment group were subjected to nociception analysis in accordance with the method described in the above section "1-4"; the concentrations of IL-6 in serum and in joint fluid were measured in accordance with the method described in the above section "1-6"; and synovitis was evaluated using IFP inflammation scores while cartilage degeneration was evaluated using Mankin scores, in accordance with the methods described in the above section "1-7".

As a result, both the pain threshold and the 50% pain threshold were significantly elevated in the MIA-OA model rats of the ASCL group compared with the MIA-OA model rats of the ADSC group (see Figure 7). Both the IL-6 concentrations in serum and in joint fluid were decreased in the MIA-OA model rats of the ASCL group compared with the MIA-OA model rats of the ADSC group (see Figure 8). Both the IFP inflammation score and the Mankin score were decreased in the MIA-OA model rats of the ASCL group compared with the MIA-OA model rats of the ADSC group (see Figure 9).

These results indicate that the therapeutic effect of ASCL on OA is markedly excellent as compared with OA treatment using ADSCs as a conventional method.

### Industrial Applicability

The present invention can provide a therapeutic agent using a specific mesenchymal stem cell line derived from an adipose tissue (ASCL), the therapeutic agent (i) stably having an excellent therapeutic effect on one or more diseases selected from knee osteoarthritis, traumatic cartilage defect, and osteochondritis dissecans, (ii) loading less physical burden on a patient, (iii) requiring no restriction on patient's hospital visit management, and (iv) having no restriction on the number of treatments.

## Claims

1. A therapeutic agent for one or more diseases selected from knee osteoarthritis, traumatic cartilage defect, and osteochondritis dissecans, the therapeutic agent comprising a mesenchymal stem cell line derived from an adipose tissue as an active ingredient, the mesenchymal stem cell line being produced by a production method comprising the following steps (A) and (B):
(A) a step of inducing differentiation of one or more cells selected from a stromal vascular fraction of a vertebrate animal adipose tissue comprising a mesenchymal stem cell, an adipose progenitor cell, and a stromal cell into a mature adipocyte; and
(B) a step of inducing dedifferentiation of the mature adipocyte obtained in step (A) to obtain a mesenchymal cell line derived from the vertebrate animal adipose tissue.

2. The therapeutic agent according to claim 1, wherein the step of inducing differentiation of one or more cells into a mature adipocyte in step (A) is a step of culturing the one or more cells in basal medium for mesenchymal cell culture comprising one or more adipose cell differentiation inducing substances selected from the group consisting of dexamethasone, isobutylmethylxanthine, insulin, and serum.

3. The therapeutic agent according to claim 1, wherein the dedifferentiation induction of the mature adipocyte in step (B) is performing ceiling culture of the mature adipocyte.

4. The therapeutic agent according to claim 1, wherein the one or more cells are cells obtained by removing the mature adipocyte from a cell population obtained by treating the vertebrate animal adipose tissue with an enzyme capable of dispersing the vertebrate animal adipose tissue cells.

5. The therapeutic agent according to claim 4, wherein the enzyme capable of dispersing the vertebrate animal adipose tissue cells is one or more enzymes selected from the group consisting of collagenase, trypsin, caseinase, clostripain, trypsin-EDTA, dispase, thermolysin, pronase, hyaluronidase, pancreatin, elastase, and papain.

6. The therapeutic agent according to claim 1, wherein the mesenchymal stem cell line derived from an adipose tissue expresses one or more surface markers selected from the following surface marker group of mesenchymal cells, and does not express one or more surface markers selected from the following surface marker group of blood cells:
surface marker group of mesenchymal cells: CD13, CD29, CD44, CD71, CD73, CD90, CD105, CD166, HLA-ABC;
surface marker group of blood cells: CD11b, CD14, CD19, CD34, CD41, CD42b, CD45, CD56, HLA-DR.

7. The therapeutic agent according to any one of claims 1 to 6, wherein the therapeutic agent is intra-articularly administered to a vertebrate animal in need of treatment of one or more diseases selected from knee osteoarthritis, traumatic cartilage defect, and osteochondritis dissecans.

8. The therapeutic agent according to claim 1, wherein the vertebrate animal is a human.
